# EUROPEAN PATENT SPECIFICATION

(11) **EP 1 121 935 B1**
(45) Date of publication and mention of the grant of the patent: **13.08.2008**
(21) Application number: 01102385.0
(22) Date of filing: 02.02.2001
(51) Int. Cl.: A61K 38/11, A61K 9/08, A61K 9/12

(54) **Pharmaceutical composition containing a small or medium size peptide**
Arzneimittel, welches ein Peptid kleiner oder mittlerer Grösse enthält
Composition pharmaceutique contenant un peptide de taille petite ou moyenne

(30) Priority: 04.02.2000 EP 00102429
(43) Date of publication of application: 08.08.2001
(60) Divisional application: 07122352.3
(73) Proprietor: Patents Exploitation Company B.V., 1082 MD Amsterdam (NL)
(72) Inventor: Woodrow, Wayne, Toronto, Ontario M1S 1P8 (CA)
(74) Representative: Gervasi, Gemma

(56) References cited:
- EP-A- 0 111 841
- EP-A- 0 346 501
- WO-A-95/01185
- WO-A-97/48379
- V. HELM ET AL.: "Stabilität von Gonadorelin und Triptorelin in wässriger Lösung" ACTA PHARMACEUTICA TECHNOLOGICA, vol. 36, no. 1, April 1990 (1990-04), page 31s XP002140992 Stuttgart (DE)
- DATABASE WPI Week 8630 Derwent Publications Ltd., London, GB; AN 1986-193349 XP002140993 & JP 61 126014 A (TEIJIN LTD), 13 June 1986 (1986-06-13)

## Description

### CROSS-REFERENCE TO RELATED APPLICATIONS

This application claims priority from EP 00 102 429.8 filed on February 4, 2000.

### FIELD OF THE INVENTION

The present invention relates to a pharmaceutical composition containing a small or medium size peptide, preferably a small or medium size cyclic peptide, free from preservatives and anyway stable before and during the use.

### STATE OF THE ART

A remarkable number of peptides, derivatives and analogues thereof are known in therapy. They are often endowed with an utterly powerful biologic activity, therefore even very small amounts are required for therapeutic goals. Among these, small and medium size peptides, preferably small or medium size cyclic peptides, more preferably those containing one or more sulfur atoms within the cyclus, and most preferably those containing at least two sulfur atoms within the cyclus, such as, for example, analogues and derivatives of oxitocin and vasopressin, such as desmopressin (termed 1-deamino-8-D-arginin-vasopressin or 1-(3-mercaptopropanoic acid)-8-D-argininevasopressin), a powerful antidiuretic useful in the treatment of urinary disorders associated to, for example, insipidus diabetes and nocturnal enuresis.

One of the problems arising with peptide drugs, especially those containing easily oxidizable sulfur bonds or sulfur bridges, is the easy degradation of their aqueous solutions.

A further problem often encountered with the preparation of pharmaceutically acceptable solutions of small and medium size peptides is a seeming loss of titre in time which is due to ongoing adsorption of the peptide drug on the surface of the container material, thus entailing a considerable loss of potency and/or activity of the pharmaceutical composition. Such adsorption problem of peptide drugs is well-known in the pharmaceutical arts. For example, calcitonin may be quickly adsorbed onto the plastic of intravenous giving sets such that addition of a protein as a "preservative" (acting as an adsorption inhibitor) is required (Martindale's "Complete Drug Reference", Pharmaceutical Press, 1999, pp.735-736). As far as e.g. insulin is concerned, it has been reported by C. Petty and N.L. Cunningham (Anaesthesiology 40, 400, 1974, recently reviewed by A.T. Florence and D. Attwood in "Physicochemical Principles of Pharmacy", third edition, pp. 328-331) that losses of as many as 78.8% of activity/potency may occur because of adsorption of insulin to glass bottles and plastic tubing used in giving sets.

Accordingly, many preservatives able of inhibiting adsorption of peptide drugs onto container materials have been investigated. For example, the patent application WO 95/01185 (in the name of Ferring AB) claims a pharmaceutical composition for administering peptides, such as desmopressin, containing a buffer, a quaternary amine as preservative or disinfectant, and an agent for controlling the osmotic pressure. Besides the preservative or disinfectant activity already cited (i.e. the prevention of the degradation of the active principle), the quaternary amine is capable of preventing the active principle to be adsorbed by the walls of the composition container, especially when these walls are of polymeric material. In fact, Example 5 shows that desmopressin solutions free from preservative lose about the half of active principle because of the adsorption by the walls of polystyrene, polypropylene and glass tubes, after 24 hours at room temperature.

The preferred quaternary amine according to WO 95/01185 is benzalkonium chloride. Recently, Hofmann T. et al., Springer-Verlag, 1998, 46:146-151 reported that this preservative causes the irreversible suppression of the nasal ciliar motility, such that its banning from the formulations for nasal administration is suggested.

The patent application WO 95/01183 (in the name of Ferring AB) discloses a composition for the nasal administration of desmopressin. Though claim 1 does not report the presence of specific excipients, it is apparent from the description and the examples that such composition, in particular the "long shelf life composition" always contains a preservative such as chlorobutanol or benzalkonium chloride.

### SUMMARY OF THE INVENTION

It has been now surprisingly found that pharmaceutical compositions containing a small or medium size peptide and which are free from preservatives not only are suitably stable (as far as degradation is concerned), likewise analogous compositions containing this kind of additive, but also do not show the problem of the adsorption of the active principle by the walls of the container, envisaged by the prior art.

### DESCRIPTION OF THE INVENTION

The present invention relates to a pharmaceutical composition as defined in claim 1. In particular, the pharmaceutical compositions of the invention, unlike test compositions prepared for clinical trials or for short term potency investigations on laboratory scale, are intended as marketable, ready-to-use products exhibiting an extended shelf life, even at room temperature.

The term "preservatives", in the context of the present application, embraces all those additives which preserve the peptide drug's titre in the pharmaceutical solution and thus prevent losses of the solution's potency and/or activity. In particular, the term "preservatives" embraces both, degradation inhibitors (like antioxidants or antimicrobial additives) as well as adsorption inhibitors (preventing adsorption of the active principle onto container walls).

The peptide of the composition of the present invention is selected from the group consisting of derivatives and analogues of oxitocin and vasopressin such, as, for example, terlipressin [(N-α-triglycin-8-lysin)-vasopressin], carbetocin [(1-desamino-1 monocarba-2(O-methyl)tyrosine)-oxitocin], and desmopressin (1-deamino-8-D-arginin-vasopressin or 1-(3-mercaptopropanoic acid)-8-D-argininevasopressin), and the salts thereof. Among the foregoing most preferred peptides, particularly preferred for the aim of the present invention are the vasopressin analogues, more in particular those analogues containing a mercaptopropanyl radical, desmopressin acetate hydrate being the most preferred.

In a preferred embodiment, the composition of the present invention has a pH comprised between 3.5 and 6. For maintaining such a pH value the composition shall contain a suitable buffer such as, for example, citric acid / disodium phosphate dihydrate or citric acid/trisodium citrate dihydrate.

The composition of the present invention may also contain an agent for controlling the osmolarity such as, for example, sodium chloride.

In a preferred embodiment, the composition of the invention contains at least 0.02 mg of desmopressin, at least 3 mg of a buffer, an amount of agent for controlling the osmolarity such as the osmolarity is maintained to the physiologic values of the human plasma, and 1 ml of purified water.

Preferably the composition of the present invention contains from 3 to 6 mg of the citric acid/disodium phosphate dihydrate buffer, or from 5 to 11 mg of the citric acid/trisodium citrate dihydrate buffer.

More preferably, the composition of the present invention contains from 0.02 to 0.15 mg of desmopressin, preferably 0.1 mg, from 1 to 2.5 mg of citric acid monohydrate, preferably 1.7 mg, from 2 to 5 mg of disodium phosphate dihydrate, preferably 3 mg, 1 ml of water and an amount of sodium chloride such that the osmolarity is kept at the physiological values of the human plasma.

The advantages provided by the composition of the present invention over the prior art compositions are apparent. The possibility of avoiding the use of preservatives has a positive rebound from the toxicological point of view as these substances - and the case of benzalkonium chloride is epitomising - are often a source of allergic and irritative reactions from the mucosae. Furthermore the present composition represents an overcoming of a prior art prejudice attesting that peptide solutions in general and desmopressin solutions in particular free from preservatives have the drawback -further to possible degradation- of suffering an adsorption process of the active principle by the container walls. As it is demonstrated below, the composition of the invention, though free from preservatives, does not show such a drawback.

The composition of the present invention is prepared in pre-sterile environment and sterilely filtered through 0,22 µm filters.

This is administered by a spray device filled in sterile environment under nitrogen. The vial of the spray device is preferably of glass or of plastic, e.g. of a polymeric material. Such device is equipped with a multidose pump of a kind allowing the prevention of the bacterial contamination of the drug solution, before and during the use, thanks to the protection of the aspiration air by an absolute filter and an auto-blocking mechanism of the actuator. An example of a spray device of this kind is that described by the patent application EP 0 815 946 (in the name of Pfeiffer GmbH Erich).

Examples of accomplishment of the present invention will be now provided.

### EXAMPLE 1

### Solution at pH 5

| | |
|---|---|
| Desmopressin acetate hydrate | 0.1 mg |
| (equal to desmopressin base) | (89 µg) |
| Citric acid monohydrate | 1.7 mg |
| Disodium phosphate dihydrate | 3.0 mg |
| Sodium chloride | 7.5 mg |
| Purified water | 1 ml |

### EXAMPLE 2

### Solution at pH 4

| | |
|---|---|
| Desmopressin acetate hydrate | 0.1 mg |
| (equal to desmopressin base) | (89 µg) |
| Citric acid monohydrate | 4.64 mg |
| Trisodium citrate dihydrate | 4.6 mg |
| Sodium chloride | 6.8 mg |
| Purified water | 1 ml |

### EXAMPLE 3

Evaluation of the adsorption of the active principle by the container walls

The compositions of Examples 1 and 2 were put in glass containers closed with a polymeric material pump, at room temperature for 4 days, and the titre in active principle was then evaluated.

The results are set forth in the following Table 1.

**Table 1**

| Composition | Titre at time zero | Titre after 4 days |
|---|---|---|
| Example 1 | 108.5% | 106.9% |
| Example 2 | 103.3% | 101.5% |

The results set forth by the table above clearly show that for both the compositions the adsorption process of the active principle on the glass and the polymeric material pump is negligible.

### EXAMPLE 4

Evaluation of the adsorption of the active principle by the container walls

The compositions of Examples 1 and 2 were put in polymeric containers closed with a polymeric material pump, at room temperature for 4 days, and the titre in active principle was then evaluated.

The results are set forth in the following Table 2.

**Table 2**

| Composition | Titre at time zero | Titre after 4 days |
|---|---|---|
| Example 1 | 103.6% | 101.6% |
| Example 2 | 101.1% | 101.1% |

The results set forth by the table above clearly show that for both the compositions the adsorption process of the active principle on the the polymeric material of both, container and pump is negligible.

### EXAMPLE 5

### Stability test

The quality attributes required for the compositions of the present invention are the following:

| Parameter | Attribute |
|---|---|
| PH | 3.5-6.0 |
| Desmopressin acetate hydrate | 90-110 µg/ml |
| Microbiological quality | Sterile |

The compositions of the present invention were evaluated by stability tests in type I glass vials equipped with dispenser pump.

The applied test protocols were the following:

### Real time test

It was carried out at a temperature of 5°C±3°C according to the scheme of Table 3.

**Table 3**

| Test | Beginni ng | 3 months | 6 months | 12 months | 18 months |
|---|---|---|---|---|---|
| PH | × | × | × | × | × |
| Desmopressin Acetate hydrate | × | × | × | × | × |
| Microbiological quality | × | - | - | - | × |

The results are set forth in the following Tables 4 and 5

**Table 4**

| (composition of Example 1) | | | | | |
|---|---|---|---|---|---|
| Test | Beginni ng | 3 months | 6 months | 12 months | 18 months |
| PH | 5.12 | 5.16 | 5.14 | 5.10 | 5.15 |
| Desmopressin Acetate hydrate | 108.3% | 107.6% | 106.4% | 107.1% | 104.7% |
| Microbiological quality | Sterile | - | - | - | Sterile |

**Table 5**

| (composition of Example 2) | | | | | |
|---|---|---|---|---|---|
| Test | Beginni ng | 3 months | 6 months | 12 months | 18 months |
| PH | 4.00 | 4.00 | 4.00 | 3.98 | 4.02 |
| Desmopressin Acetate hydrate | 109.0% | 108.1% | 108.8% | 107.7% | 106.0% |
| Microbiological quality | Sterile | - | - | - | Sterile |

### Room temperature tests

They were carried out at a temperature of 25°C±2°C and 60%±5% of relative humidity according to the scheme of Table 6

**Table 6**

| Test | Beginni ng | 3 months | 6 months | 12 months | 18 months |
|---|---|---|---|---|---|
| PH | × | × | × | × | × |
| Desmopressin Acetate hydrate | × | × | × | × | × |
| Microbiological quality | × | - | - | - | × |

The results were set forth in the following Tables 7 and 8.

**Table 7**

| (composition of Example 1) | | | | | |
|---|---|---|---|---|---|
| Test | Beginni ng | 3 months | 6 months | 12 months | 18 months |
| PH | 5.12 | 5.16 | 5.12 | 5.10 | 5.08 |
| Desmopressin Acetate hydrate | 108.3% | 104.4% | 100.8% | 98.9% | 93.0% |
| Microbiological quality | Sterile | - | - | - | Sterile |

**Table 8**

| (composition of Example 2) | | | | | |
|---|---|---|---|---|---|
| Test | Beginni ng | 3 months | 6 months | 12 months | 18 months |
| PH | 4.00 | 3.98 | 3.98 | 3.95 | 3.97 |
| Desmopressin Acetate hydrate | 109.0% | 105.8% | 100.1% | 96.9% | 92.5% |
| Microbiological quality | Sterile | - | - | - | Sterile |

The results of the tables above show how the compositions of the present invention, though free from the preservatives deemed necessary by the prior art, anyway reveal to be stable in that concerns the active principle both at low temperature (5°C) and at room temperature (25°C).

## Claims

1. A stable pharmaceutical composition having an extended shelf-life at room temperature in the form of an aqueous solution containing a therapeutically effective amount of a peptide or of a pharmaceutically acceptable derivative thereof selected from the group consisting of derivatives and analogues of oxytocin and vasopressin and the salts thereof, and containing a buffer, the said solution being free from adsorption inhibitors preventing adsorption of the active principle onto container walls and free from antioxidants and antimicrobial additives, proviso that the composition is not prepared by the embodiment wherein 40g of hydroxypropylcellulose (viscosity 1000-2000cp at 20°C in 2% water solution) and 1.5g of desmopressin acetate into 10 ml of an isotonic acetate buffer are dissolved, a homogeneous solution having a viscosity of approximately 10cp at 25°C is obtained, and the obtained water solution contains 150 µg/ml of desmopressin acetate.

2. A pharmaceutical composition according to claim 1 consisting of water, a therapeutically effective amount of a peptide or of a pharmaceutically acceptable derivative thereof selected from the group consisting of derivatives and analogues of oxytocin and vasopressin and the salts thereof, and a buffer.

3. A pharmaceutical composition according to claim 1 or 2, wherein the peptide is selected from the group consisting of the analogues of vasopressin, and the salts thereof.

4. A pharmaceutical composition according to claim 3, wherein the analogue of vasopressin contains a mercaptopropanoyl radical.

5. A pharmaceutical composition according to claim 4, wherein the analogue of vasopressin is desmopressin acetate hydrate.

6. A pharmaceutical composition according to claim 1 or 2, the employed aqueous solution having a pH comprised between 3.5 and 6.

7. A pharmaceutical composition according to claim 6, wherein the buffer is selected from the group consisting of citric acid/disodium phosphate dihydrate and citric acid/trisodium citrate dihydrate.

8. A pharmaceutical composition according to claim 1 or 2, wherein the aqueous solution contains an agent for controlling the osmolarity.

9. A pharmaceutical composition according to claim 8, wherein the agent for controlling the osmolarity is sodium chloride.

10. A pharmaceutical composition according to claim 1 or 2, the aqueous solution containing at least 0.02 mg of desmopressin, at least 3 mg of the buffer, and an amount of an agent for controlling the osmolarity such that the osmolarity is kept at the physiologic values of the human plasma, and 1 ml of purified water.

11. A pharmaceutical composition according to claim 10, the aqueous solution containing from 3 to 6 mg of citric acid/disodium phosphate dihydrate buffer, or from 5 to 11 mg of citric acid/trisodium citrate dihydrate buffer.

12. A pharmaceutical composition according to claim 11, the aqueous solution containing from 0.02 to 0.15 mg of desmopressin, from 1 to 2.5 mg of citric acid monohydrate, from 2 to 5 mg of disodium phosphate dihydrate, 1 ml of water and an amount of sodium chloride such that the osmolarity is kept at the physiologic values of the human plasma.

13. A pharmaceutical composition according to claim 1 o 2, the aqueous solution containing 0.1 mg of desmopressin, 1.7 mg of citric acid monohydrate, 3 mg of disodium phosphate dihydrate, 1 ml of water and an amount of sodium chloride such that the osmolarity is kept at the physiologic values of the human plasma.

14. A process for preparing a pharmaceutical composition according to claim 1 or 2, comprising the steps of operating in pre-sterile environment, sterilely filtrating through 0,22 µm filters, collecting the filtrate in sterile environment and distributing it in sterile vials.

15. Spray unit containing a stable pharmaceutical composition according to claim 1 or 2, and equipped with a multidose pump, absolute filter for the aspiration air, and an auto-blocking mechanism of the actuator.

16. Spray unit according to claim 15, wherein the vial is of glass.

17. Spray unit according to claim 15, wherein the vial is of plastic.

## Patentansprüche

1. Stabile pharmazeutische Zusammensetzung mit einer verlängerten Haltbarkeit bei Raumtemperatur in Form einer wäßrigen Lösung, enthaltend eine therapeutisch wirksame Menge eines Peptids oder eines pharmazeutisch akzeptablen Derivats davon, ausgewählt aus der Gruppe bestehend aus Derivaten und Analoga von Oxytocin und Vasopressin und Salzen davon und enthaltend einen Puffer, wobei die Lösung frei ist von Adsorptionsinhibitoren, die die Adsorption der aktiven Bestandteile an die Behälterwand verhindern, und frei von Antioxidantien und antimikrobischen Additiven ist, mit der Maßgabe, daß die Zusammensetzung nicht nach der Ausführungsform hergestellt ist, bei der 40 g Hydroxypropylcellulose (Viskosität 1.000-2.000 cp bei 20°C in 2 % wäßriger Lösung) und 1,5 g Desmopressinacetat in 10 ml eines isotonischen Acetatpuffers gelöst werden, eine homogene Lösung mit einer Viskosität von ca. 10 cp bei 25°C erhalten wird und die erhaltene Wasserlösung 150 µg/ml an Desmopressinacetat enthält.

2. Pharmazeutische Zusammensetzung gemäß Anspruch 1 bestehend aus Wasser, einer therapeutisch wirksamen Menge eines Peptids oder eines pharmazeutisch akzeptablen Derivats davon, ausgewählt aus der Gruppe bestehend aus Derivaten und Analoga von Oxytocin und Vasopressin und Salzen davon, und einem Puffer.

3. Pharmazeutische Zusammensetzung gemäß Anspruch 1 oder 2, wobei das Peptid ausgewählt ist aus der Gruppe bestehend aus den Analoga von Vasopressin und Salzen davon.

4. Pharmazeutische Zusammensetzung gemäß Anspruch 3, wobei das Analogon von Vasopressin ein Mercaptopropanoylradikal enthält.

5. Pharmazeutische Zusammensetzung gemäß Anspruch 4, wobei das Analogon von Vasopressin Desmopressinacetathydrat ist.

6. Pharmazeutische Zusammensetzung gemäß Anspruch 1 oder 2, wobei die verwendete wäßrige Lösung einen pH-Wert von zwischen 3,5 und 6 besitzt.

7. Pharmazeutische Zusammensetzung gemäß Anspruch 6, wobei der Puffer ausgewählt ist aus der Gruppe bestehend aus Zitronensäure/Dinatriumphosphatdihydrat und Zitronensäure/Trinatriumcitratdihydrat.

8. Pharmazeutische Zusammensetzung gemäß Anspruch 1 oder 2, wobei die wäßrige Lösung ein Mittel zur Regulierung der Osmolarität enthält.

9. Pharmazeutische Zusammensetzung gemäß Anspruch 8, wobei das Mittel zur Regulierung der Osmolarität Natriumchlorid ist.

10. Pharmazeutische Zusammensetzung gemäß Anspruch 1 oder 2, wobei die wäßrige Lösung mindestens 0,02 mg Desmopressin, mindestens 3 mg Puffer und eine Menge eines Mittels zur Regulierung der Osmolarität, so daß die Osmolarität auf den physiologischen Werten von Humanplasma gehalten wird, und 1 ml an gereinigtem Wasser enthält.

11. Pharmazeutische Zusammensetzung gemäß Anspruch 10, wobei die wäßrige Lösung von 3 bis 6 mg Zitronensäure/Dinatriumphosphatdihydrat-Puffer oder 5 bis 11 mg Zitronensäure/Trinatriumcitratdihydrat-Puffer enthält.

12. Pharmazeutische Zusammensetzung gemäß Anspruch 11, wobei die wäßrige Lösung 0,02 bis 0,15 mg Desmopressin, 1 bis 2,5 mg Zitronensäuremonohydrat, 2 bis 5 mg Dinatriumphosphatdihydrat, 1 ml Wasser und eine Menge an Natriumchlorid enthält, so daß die Osmolarität auf den physiologischen Werten von Humanplasma gehalten wird.

13. Pharmazeutische Zusammensetzung gemäß Anspruch 1 oder 2, wobei die wäßrige Lösung 0,1 mg Desmopressin, 1,7 mg Zitronensäuremonohydrat, 3 mg Dinatriumphosphatdihydrat, 1 ml Wasser und eine Menge an Natriumchlorid enthält, so daß die Osmolarität auf den physiologischen Werten von Humanplasma gehalten wird.

14. Verfahren zur Herstellung einer pharmazeutischen Zusammensetzung gemäß Anspruch 1 oder 2, umfassend die Schritte: Arbeiten in pre-steriler Umgebung, Sterilfiltrieren durch 0,22 µm Filter, Einsammeln des Filtrats in einer sterilen Umgebung und Verteilen dieses in sterile Fläschchen.

15. Sprüheinheit, enthaltend eine stabile pharmazeutische Zusammensetzung gemäß Anspruch 1 oder 2 und ausgestattet mit einer Multidosierpumpe, einem Absolutfilter zur Ansaugung von Luft und einem Autoblockiermechanismus des Auslösers.

16. Sprüheinheit gemäß Anspruch 15, wobei das Fläschchen aus Glas ist.

17. Sprüheinheit gemäß Anspruch 15, wobei das Fläschchen aus Plastik ist.

## Revendications

1. Composition pharmaceutique stable ayant une demi-vie prolongée à la température ambiante sous la forme d'une solution aqueuse contenant une quantité thérapeutiquement efficace d'un peptide ou d'un dérivé pharmaceutiquement acceptable de celui-ci choisi dans le groupe constitué de dérivés et d'analogues d'oxytocine et de vasopressine et des sels de ceux-ci, et contenant un tampon, ladite solution étant dépourvue d'inhibiteurs de l'adsorption empêchant l'adsorption du principe actif sur les parois du récipient et dépourvue d'antioxydants et d'additifs antimicrobiens, à condition que la composition ne soit pas préparée par le mode de réalisation dans lequel 40 g d'hydroxypropylcellulose (viscosité de 1000 à 2000 cP à 20°C dans une solution aqueuse à 2 %) et 1,5 g d'acétate de desmopressine dans 10 ml d'un tampon acétate isotonique sont dissous, une solution homogène ayant une viscosité d'approximativement 10 cP à 25°C est obtenue et la solution aqueuse obtenue contient 150 µg/ml d'acétate de desmopressine.

2. Composition pharmaceutique selon la revendication 1 constituée d'eau, d'une quantité thérapeutiquement efficace d'un peptide ou d'un dérivé pharmaceutiquement acceptable de celui-ci choisi dans le groupe constitué de dérivés et d'analogues d'oxytocine et de vasopressine et des sels de ceux-ci, et d'un tampon.

3. Composition pharmaceutique selon la revendication 1 ou 2, dans laquelle le peptide est choisi dans le groupe constitué des analogues de la vasopressine et des sels de ceux-ci.

4. Composition pharmaceutique selon la revendication 3, dans laquelle l'analogue de la vasopressine contient un radical mercaptopropanoyle.

5. Composition pharmaceutique selon la revendication 4, dans laquelle l'analogue de la vasopressine est l'acétate de desmopressine hydraté.

6. Composition pharmaceutique selon la revendication 1 ou 2, la solution aqueuse employée ayant un pH compris entre 3,5 et 6.

7. Composition pharmaceutique selon la revendication 6, dans laquelle le tampon est choisi dans le groupe constitué d'acide citrique/phosphate disodique dihydraté et d'acide citrique/citrate trisodique dihydraté.

8. Composition pharmaceutique selon la revendication 1 ou 2, dans laquelle la solution aqueuse contient un agent pour contrôler l'osmolarité.

9. Composition pharmaceutique selon la revendication 8, dans laquelle l'agent pour contrôler l'osmolarité est le chlorure de sodium.

10. Composition pharmaceutique selon la revendication 1 ou 2, la solution aqueuse contenant au moins 0,02 mg de desmopressine, au moins 3 mg du tampon et une quantité d'un agent pour contrôler l'osmolarité de telle manière que l'osmolarité est maintenue au niveau des valeurs physiologiques du plasma humain, et 1 ml d'eau purifiée.

11. Composition pharmaceutique selon la revendication 10, la solution aqueuse contenant de 3 à 6 mg de tampon acide citrique/phosphate disodique dihydraté ou de 5 à 11 mg de tampon acide citrique/ citrate trisodique dihydraté.

12. Composition pharmaceutique selon la revendication 11, la solution aqueuse contenant de 0,02 à 0,15 mg de desmopressine, de 1 à 2,5 mg d'acide citrique monohydraté, de 2 à 5 mg de phosphate disodique dihydraté, 1 ml d'eau et une quantité de chlorure de sodium de telle manière que l'osmolarité est maintenue au niveau des valeurs physiologiques du plasma humain.

13. Composition pharmaceutique selon la revendication 1 ou 2, la solution aqueuse contenant 0,1 mg de desmopressine, 1,7 mg d'acide citrique monohydraté, 3 mg de phosphate disodique dihydraté, 1 ml d'eau et une quantité de chlorure de sodium de telle manière que l'osmolarité est maintenue au niveau des valeurs physiologiques du plasma humain.

14. Procédé de préparation d'une composition pharmaceutique selon la revendication 1 ou 2, comprenant les étapes consistant à opérer dans un environnement pré-stérile, stériliser par filtration à travers des filtres de 0,22 µm, recueillir le filtrat dans un environnement stérile et le distribuer dans des flacons stériles.

15. Unité de pulvérisation contenant une composition pharmaceutique stable selon la revendication 1 ou 2, et équipée d'une pompe multidoses, d'un filtre absolu pour l'air d'aspiration et d'un mécanisme autobloquant du dispositif d'actionnement.

16. Unité de pulvérisation selon la revendication 15, dans laquelle le flacon est de verre.

17. Unité de pulvérisation selon la revendication 15, dans laquelle le flacon est de plastique.
